# EUROPEAN PATENT APPLICATION

(11) **EP 1 566 761 A2**
(43) Date of publication of application: **24.08.2005**
(21) Application number: 05250984.1
(22) Date of filing: 22.02.2005
(51) Int. Cl.: G06F 19/00

(54) **System, tools and methods for constructing interactive biological diagrams**

(30) Priority: 23.02.2004 US 784523
(71) Applicant: Agilent Technologies, Inc., Palo Alto, CA 94306 (US)
(72) Inventor: Kuchinsky, Allan J., San Francisco, CA 94127 (US); Vailaya, Aditya, Santa Clara, CA 95054 (US); Moh, David, San Francisco, CA 94131 (US); Bluvas, Peter, Amsterdam, NY 12010 (US)
(74) Representative: Tollett, Ian

(57) **Abstract**

Systems, tools, methods and recordable media for providing a visual grammar to be associated with a local format for creating interactive biological diagrams. Stencils (160) are provided to represent higher level representations of associated entities (130, 167, 169, 170) to interactions (137, 138, 139, 156, 157) and descriptively and unambiguously displayed. Additionally, information may be overlaid and compared with existing biological diagrams.

## Description

The present invention pertains to a system for manipulating biological data.

More particularly, the present invention relates to creation and manipulation of biological diagrams for interactive use with other forms of biological data.

The discovery of medicines and treatments for life-threatening diseases is often a process of piecing together a detailed understanding of the molecular basis of disease, a process of putting together and articulating the story of how genes and proteins interact with each other in biological pathways. Molecular biologists working in this area need to assimilate knowledge from a dramatically increasing amount and diversity of biological data. This explosion of data is made possible by emerging technologies, such as DNA microarrays, mass spectrometry, nuclear magnetic resonance, and quantitative polymerase chain reaction. There is also a vast amount of information in the scientific literature which the molecular biologist can use in deriving an understanding of the interactions between molecular entities.

One manner in which biologists use these experimental data and other sources of information is in an effort to piece together interpretations and form hypotheses about biological processes. Such interpretations and hypotheses constitute higher-level models of biological activity. Such models can be the basis of communicating information to colleagues, for generating ideas for further experimentation, and for predicting biological response to a condition, treatment, or stimulus.

One form of model that gained universal acceptance for representing biological activity is that of the Network, wherein biological entities and the interrelationships between them are represented as diagrammatic nodes and links, respectively. Biological networks are also commonly referred to as "pathways". The Network metaphor is very natural for representing the interactions between biological molecules; moreover, there is a rich history of graph theoretic network analysis tools from other domains, such as electrical engineering, which can be utilized to analyze the properties of biological networks. Thus, the Network metaphor is very useful not only as an aid in organizing information about biomolecular interactions, but also as a basis for predicting the effects of perturbations on a biomolecular network. To this end, there is a plethora of software systems available to help molecular biologists create and manipulate information related to biological networks. This includes, but is not limited to: tools for constructing, modifying, and/or refining biological networks, tools for inferring biological networks from experimental data and/or scientific literature, tools for visualizing experimental data in the context of biological networks, and tools for simulating the behavior of biological networks and/or analyzing their graph properties.

It should be noted that the network metaphor is often used to represent knowledge not limited to the context of molecular networks, but applicable to biological knowledge more broadly. For example, the interrelationships amongst physiological processes or amongst disease states are often represented in the biomedical literature via network diagrams.

As the biological community's knowledge of biological networks increases, we are also seeing dramatic increases in the sizes of elucidated biological networks, in their interconnectedness with other biological networks, and in the sheer number of biological networks. This explosion in complexity is difficult for users to manage. For example, it is very hard to visually inspect network diagrams of over a few hundred nodes, whereas the size of some protein/protein interaction networks may number in the thousands of nodes.

In the field of bioinformatics, there are many kinds of tools in which visual representation and manipulation of biological networks and pathways play a key role. For instance, in the area of systems biology, there exist graphical network editing tools, which serve as front ends to *in silico* modeling and simulation tools. Examples of such tools include NetBuilder (http://strc.herts.ac.uk/bio/maria/NetBuilder/) and JDesigner (http://www.cds.caltech.edu/~hsauro/JDesigner.htm). With these tools, users build up networks from single elements. This process can be tedious and error prone as networks grow larger.

There are systems in other domains for building up network diagrams' from graphical building blocks. An example of a leading general-purpose diagramming product of this nature is Visio (http://www.microsoft.com/office/visio/). Visio uses the notions of "macros" and "templates" to automate repetitive tasks. In the domain of integrated circuit design, there are tools for circuit layout that use graphical building blocks as elements. One approach is to use "parameterized cells" as building blocks. An example of this is the use of "PCells" in the Virtuoso Layout Editor product from Cadence Design Systems (http://www.cadence.com/datasheets/virtuoso layout editor.html). Neither of these systems is adapted for building biological diagrams, and therefore neither is suited for generating biological network information, such as protein-protein interaction networks, via knowledge extraction.

Fukuda and Takagi *(Bioinformatics,* Vol. 17, No. 9, 2001, pp 829-837) propose an hierarchical decomposition of signal transduction pathways as a method of structurally representing pathways in a form that can be processed readily by computers and easily understood by humans. However, hierarchical modules in their model are not parameterized, each module is a completely separate instantiation of a set of primitive entities. There is no way to "reuse" similar modules by making substitutions to a subset of the entities in a module. Thus, this method fails to take advantage of a good deal of the inherent regularity in biological networks that occurs.

In view of the existing systems, what is needed are systems methods and tools capable of not only easily and automatically generating biological diagrams based upon commonly understood sets of building blocks that reflect biological behavior, where the building blocks can be combined to create biological diagrams of more manageable complexity than networks created from distinct molecular components.

The present invention provides systems, methods and computer readable media for manipulating biological data. The present invention provides a visual grammar for biological entities and interactions, which may be used in conjunction with a local format textual grammar to link various forms of biological data for their interactive use.

A composible and extensible library of stencils, essentially a visual grammar for biological diagrams, is provided, wherein each stencil comprises graphical elements representing entities and at least one interaction, each graphical element comprising biological semantics representative of a particular type of biological entity or interaction; and slots for providing specific biological information, including specific entity names and directionality of interactions. The visual grammar is designed to accompany a local format textual grammar, enabling interactive functions to be performed among biological diagrams, textual documents and experimental data.

Stencils may be used to represent knowledge in a broad biological context, for example the interrelationships amongst physiological processes or amongst disease states, as well as bio-molecular interactions.

A tool for building biological networks of interactions is provided, which includes a network viewer, a canvas for populating stencils with entities and relationships/interactions identified, and means for selecting populated stencils, merging common entities and displaying a resulting network of the interactions in the network viewer.

Means for comparing experimental data with the resulting network, based upon means for rule checking, are further provided. Discrepancies identified between the experimental data and the resulting network may be visually identified, such as by highlighting, accentuating, or the like.

Stencils may be provided for displaying multiple levels of abstraction within a biological network. For example, multiple interactions and their associated entities may be combined to represent a higher order biological concept.

Free form extension capability is further provided, wherein a stencil may be extended by sketching or free drawing additional entities and/or interactions in linkage with the existing entities and interactions already displayed by the pre-existing stencil.

A system for manipulating biological data is provided which comprises a library of re-usable stencils for representing biological interactions; means for selecting stencils to be populated with specific biological information; means for assigning specific biological data to selected stencils; means for displaying stencils with the assigned specific biological data; and means for linking the displayed stencils with other sources of biological data from which the specific biological data was extracted, using a local formatting language.

Further described are means for connecting common elements of the stencils with assigned specific biological data to display a biological diagram having the stencils as components thereof.

Means for designing and saving additional stencils, not previously contained in the library, are further provided.

Means for designing and associating rules with the stencils are provided. Further, means for rule checking the rules to validate an interaction represented by a stencil containing specific biological data are provided. Also rule checking of the rules against additional data may be performed.

Further, means for navigating to data referenced from specific biological data and displayed on at least one of the stencils is made possible using the local format.

Two or more stencils may be compared as to the specific data assigned thereto and results of the comparison may be displayed on a viewer according to the present invention.

Using the present invention, specific biological data represented in stencils may be mapped to an existing biological diagram.

By use of the present invention, a user may easily and conveniently construct diagrammatic representations of data/text that can be used to make an interactive biological diagram. For example, the present invention includes a method of providing a stencil comprising graphical elements representing entities and at least one interaction and slots for providing specific biological information, including specific entity names and directionality of interactions; assigning specific biological information to the stencil to identify entities involved in the interaction; and interactively assigning the directionality of at least one interaction, thereby disambiguating a graphical representation of the interaction. Information used in populating the stencils may be entities and interactions identified by text mining an existing textual document, or other source of biological information.

Methods for using each of the above tools and systems, either alone or in any usable combination are also provided.

The present invention provides systems, tools and methods for providing interactive capabilities for user involvement in disambiguating biological information to be used in generating a biological diagram. For example, one such tool provides a text viewer into which at least a portion of a textual document may be imported and viewed; means for text mining the text having been imported into the text viewer; a canvas area for generating biological diagrams; at least one pre-designed blank stencil representing a particular type of interaction; and means for populating stencils on the canvas with one or more of the entities and interactions identified during text mining. The entities and interactions populating the stencils each point back to at least one location in a portion of the textual document where each was identified

A list-based text editor that lists entities and interactions having been identified by the text mining may also be provided, and means for assigning directionality to the listed interactions may be used to disambiguate within the lists. Slots are associated with each interaction listed so that a user can identify one or more of the listed entities involved in the interaction, and assign roles of each of these entities, as played in the interaction.

These and other advantages and features of the invention will become apparent to those persons skilled in the art upon reading the details of a number of preferred embodiments of the invention as more fully described below with reference to the drawings, in which:-
Fig. 1 is a schematic representation of a system for facilitating interaction, comparisons, overlays, etc. of information from different categories, such as textual material (e.g., scientific literature), experimental data and biological diagrams.
Fig. 2A shows a schematic representation of a blank stencil according to the present invention.
Fig. 2B shows the stencil of Fig. 2A after having filled values into the blank slots of the blank stencil.
Fig. 3 shows a biological network diagramming tool which includes a palette of predefined stencils that can be used in network diagramming.
Fig. 4 illustrates a conflict between a set of putative interactions and experimental data, and display of this conflict, based upon rule checking.
Fig. 5A shows an example signaling pathway broken down into hierarchical blocks.
Fig. 5B shows a mapping of stencils for an interferon-alpha mediated signal transduction pathway on the diagram of Fig. 5A.
Fig. 6 shows two examples of stencils, each as blank stencils, as well as filled-in versions.
Fig. 7shows another example of a stencil, both as a blank stencil, and as a filled-in stencil.
Fig. 8A shows an empty stencil and Fig. 8B shows the same stencil filled-in. This stencil represents a signal transduction sub-path, containing the binding of a ligand to a receptor on a cell membrane and a small number of subsequent cascading activation events.
Fig. 9 shows a screen shot of an example of an application tool in which stencils may be used to facilitate disambiguation of directionality of processes/interactions extracted from scientific literature and incorporated into a text viewer.
Fig. 10 shows the tool of Fig. 9 having a blank stencil populated in a canvas area of the tool.
Fig. 11 shows completion of the population (or "filling in") of the stencil shown in Fig. 10, along with automatic assignment of roles in the interaction list.
Fig. 12 is a block diagram illustrating an example of a generic computer system which may be used in implementing the present invention.

Before the present systems, tools and methods are described, it is to be understood that this invention is not limited to particular software, hardware, software language or symbol described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a stencil" includes a plurality of such stencils and reference to "the diagram" includes reference to one or more diagrams and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DEFINITIONS

In the present application, unless a contrary intention appears, the following terms refer to the indicated characteristics.

The term "biological diagram", as used herein, refers to any graphical image which contains depictions of concepts found in biology. Biological diagrams include, but are not limited to, pathway diagrams, cellular networks, signal transduction pathways, regulatory pathways, metabolic pathways, protein-protein interactions, interactions between molecules, compounds, or drugs, and the like.

A "biological concept" "entity" or "item" refers to any subject of interest in the biological domain, including, but not limited to proteins, genes, molecules, tissues, organs, disease processes, cellular functions, anatomical structures, physiological systems, biopolymers, nucleotides, and the like. A "biological concept", "entity" or "item" may be a subject of interest that a researcher is endeavoring to learn more about. For example, a biological concept, entity or item may be one or more genes, proteins, molecules, ligands, diseases, drugs or other compounds, textual or other semantic description of the foregoing, or combinations of any or all of the foregoing, but is not limited to these specific examples.

A "biopolymer" is a polymer of one or more types of repeating units. Biopolymers are typically found in biological systems and particularly include polysaccharides (such as carbohydrates), and peptides (which term is used to include polypeptides and proteins) and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another.

A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.. For example, a "biopolymer" includes DNA (including cDNA), RNA, oligonucleotides, and PNA and other polynucleotides, regardless of the source. An "oligonucleotide" generally refers to a nucleotide multimer of about 10 to 100 nucleotides in length, while a "polynucleotide" includes a nucleotide multimer having any number of nucleotides. A "biomonomer" references a single unit, which can be linked with the same or other biomonomers to form a biopolymer (for example, a single amino acid or nucleotide with two linking groups one or both of which may have removable protecting groups).

An "interaction" or "relation", as used herein, refers to a relationship or action that occurs between entities or nodes (nouns) and may also be referred to as a "verb" (in a local format, for example). Verbs are identified for use in the local format to construct a grammar, language or Boolean logic. Examples of verbs, but not limited to these, include upregulation, downregulation, inhibition, promotion, bind, cleave and status of genes, protein-protein interactions, drug actions and reactions, etc.

When one item is indicated as being "remote" from another, this is referenced that the two items are at least in different buildings, and may be at least one mile, ten miles, or at least one hundred miles apart.

"Communicating" information references transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

A "processor" references any hardware and/or software combination which will perform the functions required of it. For example, any processor herein may be a programmable digital microprocessor such as available in the form of a mainframe, server, or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic or optical disk may carry the programming, and can be read by a suitable disk reader communicating with each processor at its corresponding station.

"May" means optionally.

Methods recited herein may be carried out in any order of the recited events which is logically possible, as well as the recited order of events.

"Local format" refers to a restricted grammar/language used to represent extracted semantic information from diagrams, text, experimental data, etc., so that all of the extracted information is in the same format and may be easily exchanged and used in together. The local format can be used to link information from diverse categories, and this may be carried out automatically. The information that results in the local format can then be used as a precursor for application tools provided to compare experimental data with existing textual data and biological models, as well as with any textual data or biological models that the user may supply, for example.

A "node" as used herein, refers to an entity, which also may be referred to as a "noun" (in a local format, for example). Thus, when data is converted to a local format according to the present invention, nodes are selected as the "nouns" for the local format to build a grammar, language or Boolean logic.

A "link" as used herein, refers to a relationship or action that occurs between entities or nodes (nouns) and may also be referred to as a "verb" (in a local format, for example). Verbs are identified for use in the local format to construct a grammar, language or Boolean logic. Examples of verbs, but not limited to these, include upregulation, downregulation, inhibition, promotion, bind, cleave and status of genes, protein-protein interactions, drug actions and reactions, etc.

A "rule", as used herein, refers to a procedure that can be run using data related to stencils, nodes, and links. Rules can be declarative assertions that can be computationally verified, for example "an enzyme must be a protein", or they can be arbitrary procedures that can be computationally executed using data related to stencils, nodes, and links, for example "if there is a relation such that entity A activates entity B, and if A is in state activated, then set B in state activated".

A "stencil", as used herein, refers to a diagrammatic representation which may contain one or more biological concepts, entities, times, interactions, relationships and descriptions (generally, although not necessarily, graphic descriptions) of how these interact. Stencils function similarly to macros in Microsoft Word or Excel, with respect to their functionality for generating more than one node or link at a time when constructing a biological diagram. Stencils may be comprised of graphical elements, such as shapes (e.g. rectangles, ovals), lines, arcs, arrows, and/or text. These elements have biological semantics; that is, elements represent types of biological entities, such as a genes, proteins, RNA, metabolites, compounds, drugs, complexes, cell, tissue, organisms, biological relationship, disease, or the like.

A "biological network" refers to a graph representation (which may also include text, and other information) wherein biological entities and the interrelationships between them are represented as diagrammatic nodes and links, respectively. Examples of biological networks include, but are not limited to pathways and protein-protein interaction maps

A "pathway" refers to an ordered sequence of interactions in a biological network. An example of a pathway is a cascade of signaling events, such as the wnt/beta-catenin pathway, which represents the ordered sequence of interactions in a cell as a result of an outside stimulus, in this case, the binding of the wnt ligand to a receptor on the membrane of the cell. The terms "pathway" and "biological network" are sometimes used interchangeably in the art.

"Phosphorylation" refers to the addition of phosphate groups to hydroxyl groups on proteins (side chains s, T or Y) catalysed by a protein kinase often specific) with ATP as phosphate donor. Activity of proteins is often regulated by phosphorylation. Phosphorylation is one type of post-translational protein modification mechanism.

"Activated" refers to the state of a biochemical entity wherein it is enabled for performing its function.

"Inhibited" is used to refer to the state of a biochemical entity wherein it is wholly or partially disabled or deactivated for performing its function.

"Up-regulated" refers to a state of a gene wherein its production of corresponding RNA (ribonucleic acid) transcript is significantly higher than in a reference condition.

"Down-regulated" refers to refers to a state of a gene wherein its production of corresponding RNA transcript is significantly lower than in a reference condition.

A "co-factor" is an inorganic ion or another enzyme that is required for an enzyme's activity.

An effective approach to managing complexity is to use abstraction to group together sets of smaller objects into collections that can be thought of as a single entity. This reduces complexity because there is a smaller number of distinct items that one has to keep in mind when considering complex information. Stencils provide a visual biological language/grammar made up of composible patterns and motifs that have biological meaning. Stencils may be used as aggregate components of biological networks and processes. Stencils help to manage complexity by providing higher levels of abstraction than those provided by an unstructured collection of atomic elements, such as entities and interactions, nouns, verbs, genes, proteins, etc. Because grammar consists of rules, and stencils provide a visual grammar, a stencil is an embodiment of rules. Stencils may be composed of ALFA objects (i.e., using the local format, as described and referenced herein, as well as in commonly owned, co-pending Application Serial No. 10/154,524 filed May 22, 2002 and titled "System and Method for Extracting Pre-Existing Data from Multiple Formats and Representing Data in a Common Format for Making Overlays", in commonly owned, co-pending Application Serial No. 10/641,492 filed August 14, 2003 and titled "Method and System for Importing, Creating and/or Manipulating Biological Diagrams", and in commonly owned, co-pending Application Serial No. 10/642,376 filed August 14, 2003 and titled "System , Tools and Method for Viewing Textual Documents, Extracting Knowledge Therefrom and Converting the Knowledge into Other Forms of Representation of the Knowledge". Application Serial No. 10/154,524, Application Serial No. 10/641,492 and Application Serial No. 10/642,376 are each incorporated herein, in their entireties, by reference thereto.

Mapping may be performed between ALFA objects and a stencil, and vice versa. This mapping may be many-to many (i.e., many features mapped to many ALFA objects, and vice versa). In the same way, mapping may be performed between stencils and biological diagrams, between stencils and textual documents, and/or between stencils and experimental data. Existing biological diagrams may be imported, according to the present invention, and parsed into stencils, in a manner similar to that described in co-pending, commonly owned Application Serial No.10/155,675 filed May 22, 2002 and titled "System and Methods for Extracting Semantics from Images". Application Serial No. 10/155,675 is hereby incorporated herein, in its entirety, by reference thereto. Diagrams may be constructed from stencils and existing diagrams may be extended with stencils and/or with hand-drawn extensions and the like, according to the present invention.

A method and system for user-guided knowledge extraction is described in co-pending commonly owned Application Serial No. 10/154,524. Described are methods and systems wherein automated text mining techniques are used to extract "nouns" (e.g. biological entities) and "verbs" (e.g. relationships) from sentences in scientific text. Thus, knowledge extraction from scientific literature, e.g. via text mining, can identify biological entities that are involved in a relationship, for example a promotion interaction involving two genes. The resulting interpretation is represented in a restricted grammar, referred to as "local format". A software program that implements this format is the ALFA (Agilent Local Format Architecture) Text Viewer (ATV), from Agilent Technologies, Inc., Palo Alto, California, which is described in more detail in co-pending, commonly owned Application Serial No. 10/642,376 The local format serves as a structured way for the user to review and understand the essence of a scientific text. It also serves as a biological object model that can be manipulated by other computational tools.

A diagram viewer may be used to view biological diagrams, import graphical knowledge from the same and convert it to the local format for use with text and/or data. Further special features for conversion of biological diagrams, as well as construction of biological diagrams, which may be accompanied with use of the local format can be found in co-pending, commonly owned Application Serial No. 10/641,492

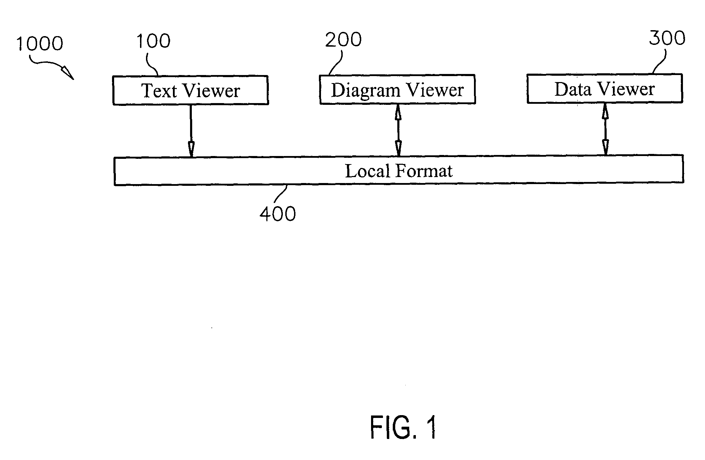

Fig. 1 is a schematic representation of a system 1000 for facilitating interaction, comparisons, overlays, etc. of information from different categories, such as textual material (e.g., scientific literature), experimental data and biological diagrams. Using a local format infrastructural layer 400 (as described in co-pending Application Serial No. 10/154,524 for example, knowledge from one representation (text, data or graphical) may be transformed to one or more other of the representations. This allows combining knowledge from different representations for comparison purposes, for constructing new and more detailed representations of knowledge, and the like. At the local format level 400 the knowledge is converted to a canonical or abstract representation. This abstract representation serves as a common language (local format) which can be used for textual representations, data representations and graphical representations of knowledge.

While many different textual editors or viewers may be used to access textual representations of knowledge and input such knowledge for conversion to the local format (some may also even data mine and automatically extract nouns and verbs, as noted above), textual viewer 100, provides for further user interaction for improvement of the knowledge gathered, as well as improvement of the accuracy when converting such knowledge. Any text mining algorithm providing an object model which can be mapped to the local format model used by the present invention may successfully interact with the tools of the present invention.

A diagram viewer 200 may be used to view biological diagrams, import graphical knowledge from the same and convert it to the local format at 400 for use with text and/or data. Further special features for conversion of biological diagrams, as well as construction of biological diagrams, which may be accompanied with use of the local format are described below. Experimental data may be imported and converted to the local format, using a data viewer 300, for overlays on textual documents, biological diagrams, or incorporation of such knowledge with textual knowledge and/or graphical knowledge, through conversion of all types to a local format. However a specific data viewer having functionality analogous to that of the text viewer 100 and diagram viewer 200 according to the present invention, and as further described in Application Serial No. 10/642,376 has not yet been developed, as the complexities in addressing specific requirements for forming relationships among individual data points and disambiguating such relationships is much more challenging than the tasks presented by either textual knowledge or diagram knowledge. Another viewer for creating and displaying interactive biological diagrams is described in co-pending, commonly owned Application Serial No. 10/641,492

Thus, the infrastructural layer 400 provides the means/data model by which knowledge from different sources may be converted and displayed at various endpoints (applications) such as text viewer 100, diagram viewer 200 and data viewer 300.

One aspect of the present invention is to provide a visual grammar, to accompany the local format, and to represent interrelationships amongst biological entities and activities. The visual grammar is based upon a library of stencils that graphically represent common types of biological entities and connections between them. The present invention also provides lightweight software tools for composing and editing the stencils, as well as tools for linking the elements of stencils, and their values, to other data elements, datasets, and the local format. Stencils may be comprised of graphical elements, such as shapes (e.g. rectangles, ovals), lines, arcs, arrows, and text. These elements have biological semantics; that is, elements represent types of biological entities, such as a genes, proteins, RNA, metabolites, compounds, drugs, complexes, cell, tissue, organisms, biological relationship, disease, or the like.

The biological semantics facilitate linking of the stencils with other forms of biological data. Further, stencils represent composites of biological activity, and therefore may function like "macros" for easier and more rapid building of biological diagrams. Stencils permit two-way interactions between textual documents and diagrams, or between diagrams and other forms of data such as experimental data, for example. Further stencils support user-controlled graphical exploration of alternatives, such as alternatives to pre-existing diagrams. Stencils may be used collaboratively among multiple users, whether by providing a blank set of stencils as a starter template, sharing of filled-in stencils, collaboratively filling in stencils, or any combination of these.

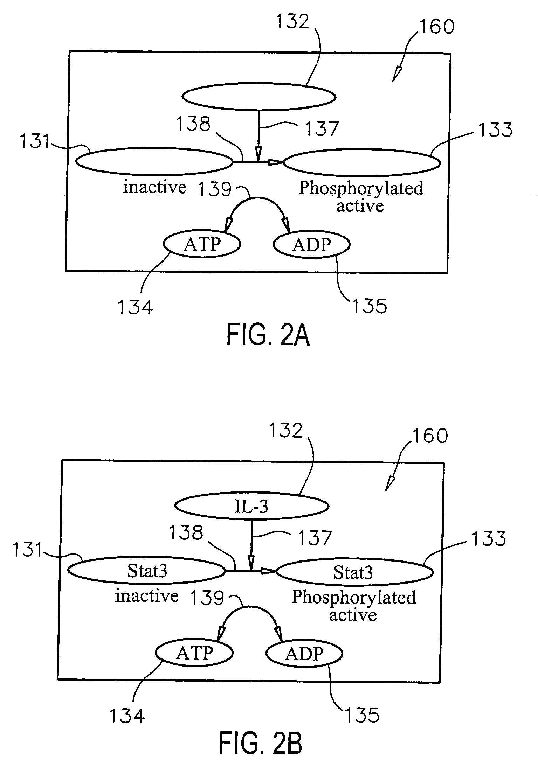

Fig. 2A shows a schematic representation of a stencil 160. This example stencil is "empty", i.e. its slots have not yet been assigned to entities. This example stencil is for a phosphorylation biochemical reaction, wherein a first entity activates a second entity via phosphorylation. This represents a very common biochemical interaction, one that is the basis for signaling in biological pathways. Ellipse 132 is a slot for the first entity. The second entity is contained in slots 131 and 133, for its inactive and active phosphorylated states, respectively. Slots 134 and 135 contain entities for reaction co-factors adenosine triphosphate (ATP) and adenosine diphosphate (ADP), respectively. Thus slots 134 and 135 contain "constants", since ATP and ADP are co-factors in all phosphorylation interactions. The interaction links 137, 138 and 139 are also constants, since the same interactions (e.g., activation, phosphorylation) occur in all phosphorylation interactions. Slots 131, 132, and 133 contain "variable" entities, which differ for different phosphorylation interactions. Slots 131 and 133 refer to the same underlying concept, and contain different states of the biological entity represented by that concept. This equivalence of concept may be enforced by rule checking, as described later.

Fig. 2B shows the "filled in" version of the phosphorylation stencil of Fig. 2A. The activating entity in slot 132, in this example, is the IL-3 protein; the entity to be activated in slot 131 and the activated entity in slot 133 is the protein Stat3.

Stencils afford the user the ability of constructing higher level representations, compared to simply constructing representations entity by entity and interaction by interaction. Figs. 2A-2B show only a simple example, as stencils may be used to represent multiple levels of abstraction within a single stencil. For example, a stencil may be provided to show molecular interaction as well as a high-level outcome, e.g. interactions leading to trauma or apoptosis. Additionally, other forms of structured experimental data and/or unstructured data may be linked to stencils accordance with the present tools and techniques. Structured data, such as experimental data may be linked using the local format, as described above. Unstructured data, such as annotations, bit map images, scans from lab notes, etc. may be linked to aid the user in understanding something about the stencil or describing certain aspects of the stencil.

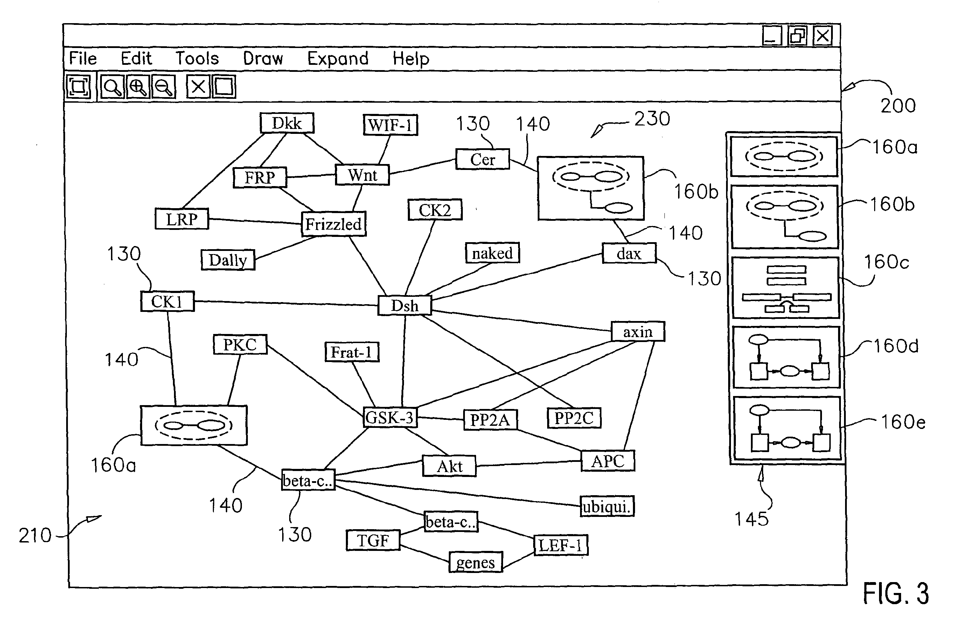

Fig. 3 shows a network diagram editing tool 200, with incorporated palette 145 of predefined stencils 160 (e.g., 160a - 160e). Stencils may be added to the network diagram 230 by dragging and dropping from stencils palette 145 onto main canvas 210 of the network editor 200. Stencils 160, when added to the network diagram 230, may be connected to components of the network diagram by using primitive "connect" operations of the diagram editing tool 200. In the preferred embodiment, a "connect" operation can be accomplished in the diagram editing tool 200 by drawing a line 140 from one of the entities 130 in the diagram 230 to an edge of the incorporated stencil 160. In Fig. 3, stencils 160a and 160b are incorporated into the network in this manner.

Slots in stencils 160 may be assigned entities in a number of ways. In the preferred embodiment, assignment of slots can be done in the diagram editor by selecting the graphical slot via double-click of mouse on the slot, then typing the name of an entity into the selected graphical slot. Another method of assigning slots in a stencil is to drag and drop a representation of an entity from another tool, such as the VistaClara exploratory data analysis tool from Agilent Technologies, Palo Alto California, and as described in co-pending, commonly assigned Application Serial No. 10/403,762 filed March 31, 2003 and titled :Methods and Systems for Simultaneous Visualization and Manipulation of Multiple Data Types". Application Serial No. 10/403,762 is incorporated herein, in its entirety, by reference thereto. Using this option, the user selects a row in the VistaClara tool and drags and drops it onto a slot in the stencil, wherein the stencil may be incorporated into a network diagram in a diagram editing tool, as described above.

As noted above, stencils may contain embedded "rule checking" so that assumptions implicit in the semantics of the stencil can be validated against actual data and facts. Each stencil may be associated with a set of logical assertion rules that can be run by the user. A rule is a procedure that can be run using data related to stencils, nodes, and links. Rules can be declarative assertions that can be computationally verified, for example "an enzyme must be a protein", or they can be arbitrary procedures that can be computationally executed using data related to stencils, nodes, and links, for example "if there is a relation such that entity A activates entity B, and if A is in state activated, then set B in state activated". In the latter example, the rules can used as the basis for generating values in simulations of biological processes.

For example, in the case of Figs. 2A and 2B, rule checking may be used to ensure that the assignments to slots 131, 132, and 133 are biologically meaningful. In the example of Fig. 2A, the entity assigned to slot 132 should be a member of the kinase family of proteins (which are agents of phosphorylation) and the entities in slots 131 and 133 should refer to the same underlying protein. Examples of other rules that can be associated with stencils are:
- Do putative promotion/inhibition relationships between genes/proteins fit the experimental data?
- Do reactions have their necessary components and preconditions?
- Do catalysts exist in enough concentration to drive the reaction in the desired direction?
- Are there "unreachable" reactions in the composed diagram?
In the preferred embodiment, rules are ALFA objects. They can be attached as attributes to a stencil. Rules apply predicate expressions over a stencil's attribute/value pairs and return a Boolean (true/false) value. An example of pseudo-code for a phosphorylation rule predicate follows:

### 1. Simple Rule Check for phosphorylation

if (this.activator.hasCategory(Category.KINASE,GO_classification tree))

### 2. Use of Rule as a Computational Procedure

if (this.activator.getPropValue("regulationState").equals
   (RegulationState.UP_REGULATED))
{ this.activated.setProp ("regulationState",
   RegulationState.UP_REGULATED);
   this.setRuleOutput (RegulationState.UP_REGULATED));}

Further, rules may have operations that may be run depending upon the success or failure of the execution of the rule predicate (i.e. whether the predicate returns TRUE or FALSE). An example of an operation is the posting of an error message to the user when a phosphorylation rule predicate fails, e.g. when the entity assigned to stencil slot 132 is NOT a kinase.

Such rules may also be composed and propagated across stencils when stencils are combined into larger diagrams. A stencil rule operation may be used to output a value, which in turn may be used as an input value for another stencil's rule checking.

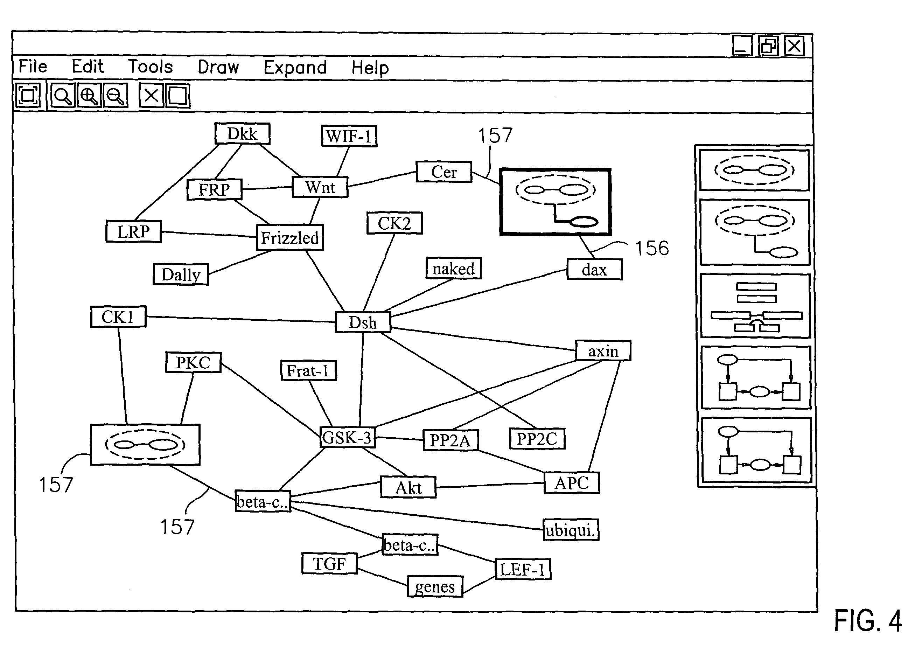

The present invention further provides the ability to build networks of interactions by composing entities, interactions, and stencils. The system merges interactions with common entities, forming a graph structure. The user may associate this network with experimental data values, performing an informal verification of the putative network against actual data. This is made possible by the inclusion of embedded rule checking in the stencils, so that assumptions implicit in the semantics of each stencil can be validated against actual data and facts. When the graph structure is created, sets of interactions that are equivalent to stencils are identified and the rules that are associated with particular stencils at issue are run against the experimental data values. The results of this verification are shown by data overlay upon the entities and interactions in the putative network. Discrepancies and contrasts may be highlighted, for example, by accentuating putative interactions that conflict with the experimental data. The results of such a comparison are shown in Fig. 4, where a discrepancy is indicated by the highlighting of interaction 156. Also, note that interactions 157 have not been highlighted, as no discrepancies have been detected. The details of the data overlay technique used can be found in co-pending, commonly assigned Application No. 10/155,616, filed May 22, 2002 and titled "System and Methods for Visualizing Diverse Biological Relationships". Application No. 10/155,616 is hereby incorporated herein, in its entirety, by reference thereto.

The present system also provides the ability to decompose graphical structures into component stencils, whether the graphical structure was previously assembled using stencils or not. If the graphical structure was previously assembled using stencils, decomposition is a simple matter, since the component stencils are already mapped via the local format. When acting upon a pre-existing graphical structure that was not previously assembled using stencils, however, the graphical structure must be converted into local format objects and then searched for sub-graphs that match stencils. The graphical structure can be converted into local format objects in a manner described in co-pending commonly owned Application Serial No. 10/641,492

Local format objects may be searched for sub-graphs that map to stencils in a manner similar to that described in Shen-Orr, S. et al, Network Motifs in the Transcriptional Regulation Network of Escherichia coli, *Nature Genetics,* 2002, which is incorporated herein, in its entirety, by reference thereto. A network of local format objects may be represented by a connectivity matrix. Possible combinations of sub-matrices of three and four nodes each are tested for equivalence with stencils in the library. Equivalence may be determined by two measures. First, the sub-matrix must be isomorphic to the stencil in the library, that is, each must have the same number of nodes and the same number of connections between connected nodes. Second, the elements of the sub-matrix must be consistent with the rules on the stencil. For example, a rule on a stencil may require that a node in a given position represent a MAP-Kinase protein. The sub-matrix must then have a MAP-Kinase protein in that given node position as a prerequisite of an equivalence finding.

Stencils may be decomposed (or partially decomposed) into component entities and interactions. Since stencils are composed of local format objects, complete or partial decomposition into component entities and interactions is a simple "ungroup" operation. Basically, the stencil instance is deleted and its components remain.

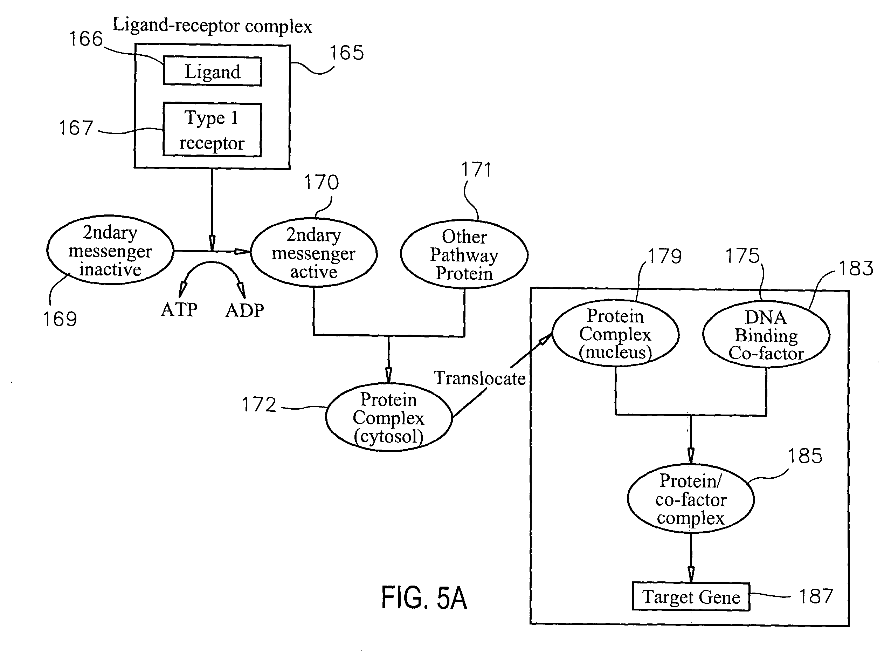

The present invention further provides the ability to compare stencils populated with extracted knowledge against an existing biological network. The user may load an existing network diagram into the system or select a subset of an existing network via search. The system overlays the populated stencils upon the imported diagram, such as by color-coding those nodes and arcs in the imported diagram that correspond to the stencils describing such entities and interactions, for example. An example of this functionality, based on an Interferon-alpha mediated signal transduction pathway imported from the SPAD Signaling Pathway Database (http://www.grt.kyushu-u.ac.jp/eny-doc/spad.html) is shown in Figs. 5A-5B. Fig. 5A shows an example signaling pathway broken down into hierarchical blocks, as per Fukuda and Takagi *(Bioinformatics,* Vol. 17, No. 9, 2001, pp 829-837). The pathway diagram of Fig. 5A depicts the reaction of a living cell, at the molecular level, to an external stimulus, ligand 166, which binds to a Type I receptor 167. This binding initiates a cascade of events resulting in the production of new proteins as the response to the initial stimulus. The binding of ligand 166 to receptor 167 forms a ligand-receptor complex 165, which in turn catalyzes a phosphorylation reaction that activates a secondary messenger inactive protein 169, forming a secondary messenger active protein 170. Secondary messenger active protein 170 then binds with other pathway protein(s) 171 to form a protein complex 172, which in turn translocates across the nuclear membrane 175 into the nucleus, where it binds with a DNA binding co-factor 183, to form a protein/co-factor complex 185. Protein/co-factor complex 185 binds to a region of DNA, and such binding activates the expression of target gene(s) 187. The expression of target gene(s) 187 results in the production of other proteins, which typically have functions related to the mechanism for responding to the initial stimulus, for example, production of cytokine proteins in response to an injury.

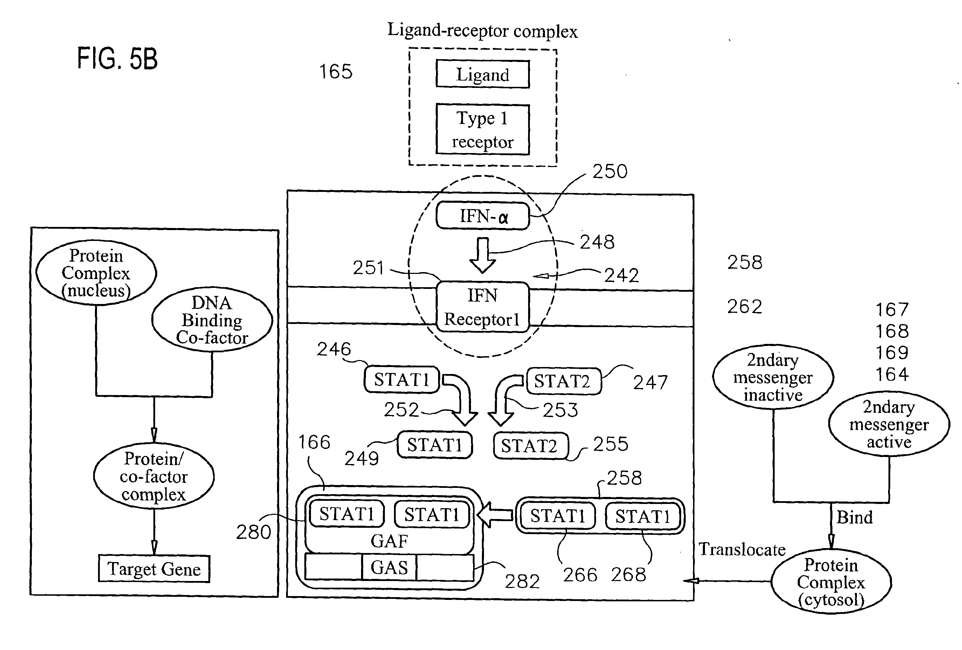

Fig. 5B illustrates a mapping of stencils for these blocks onto a signaling pathway representative of the SPAD Interferon-alpha mediated signaling pathway. Ligand-receptor complex stencil 165 maps to interactions along the membrane in the pathway between IFN-Receptor II 251 and IFN-alpha ligand 250. Secondary messenger inactive entity 167 maps to entities STAT1 246 and STAT2 247, and secondary messenger active entity 170 maps to STAT1 249 and STAT2 255.. The protein complex entity 169, formed from the binding of STAT1 and STAT2 proteins 266 and 268, translocates 164 to the nuclear regulatory complex stencil 166.

A further aspect of this overlay technique uses an automated search to search for existing networks that contain a user-specified set of interactions, such as may be contained in one or more stencils, for example. The networks found to include the specified set of interactions are then provided to the user for selection among this set to overlay the extracted interactions and entities.

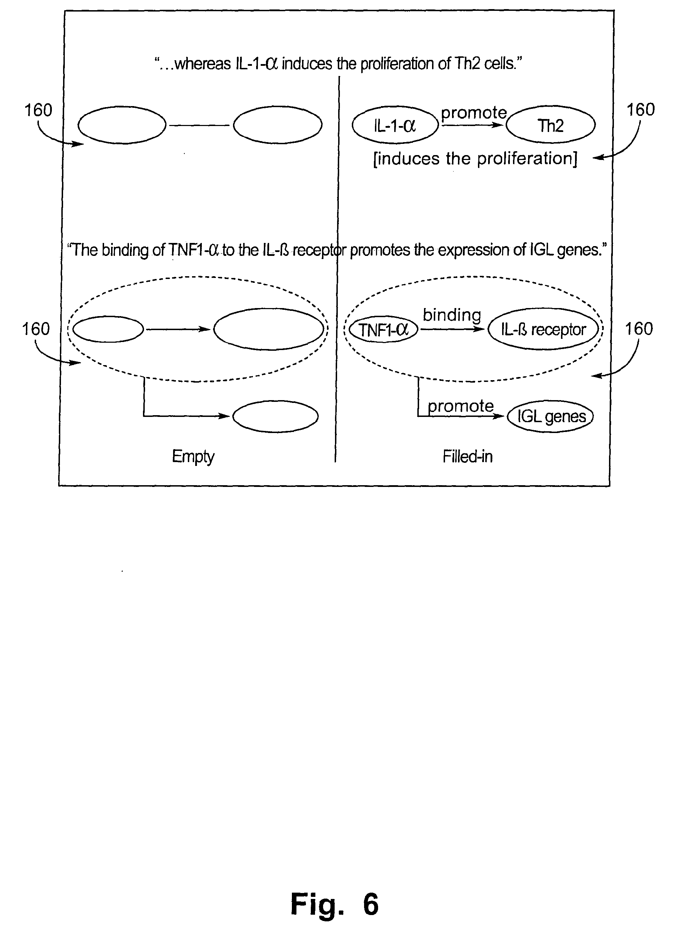

Figs. 6 - 8B show further examples of stencils according to the present invention. As noted above, stencils are used to develop a visual grammar, to accompany the local format, to represent interrelationships amongst biological entities and activities. This visual grammar is based upon a composible and extensible library of stencils that graphically represent common biological entities and connections therebetween. Each stencil includes graphical elements, such as shapes (e.g. rectangles, ovals), lines, arcs, arrows, and/or text. The stencil elements have biological semantics; that is, an element may represent a biological entity, such as a gene, protein, RNA, metabolites, compounds, drugs, complexes, cell, tissue, organism, biological relationship, or disease, etc. Each stencil contains a set of slots, each of which represents a "placeholder" for a biological entity or relation (e.g., "nouns" or "verbs").

Stencils may be abstracted by creating multiple representations of a stencil. For example, a "logical" representation of a stencil may show one or more interactions between entities, e.g., "A activates B", while a "biochemical" representation of this stencil may show "A activates B via phosphorylation". An example of a "logical" representation has been illustrated in Fig. 6 (i.e., "TNF1-alpha binds IL-beta receptor and promotes IGL Genes"). Further, stencils showing location (i.e., where in the cell the interaction occurs), time and/or interactions may be provided. Such a stencil may be further abstracted to another stencil that does not display time or location, such as a simple "logical" representation. Thus, stencils may be transformed or abstracted between simple and complex forms. Generally, if a complex stencil is defined, then a simple stencil may be constructed from such complex stencil, but not the reverse, since a simple stencil will have insufficient data to construct the complex stencil therefrom.

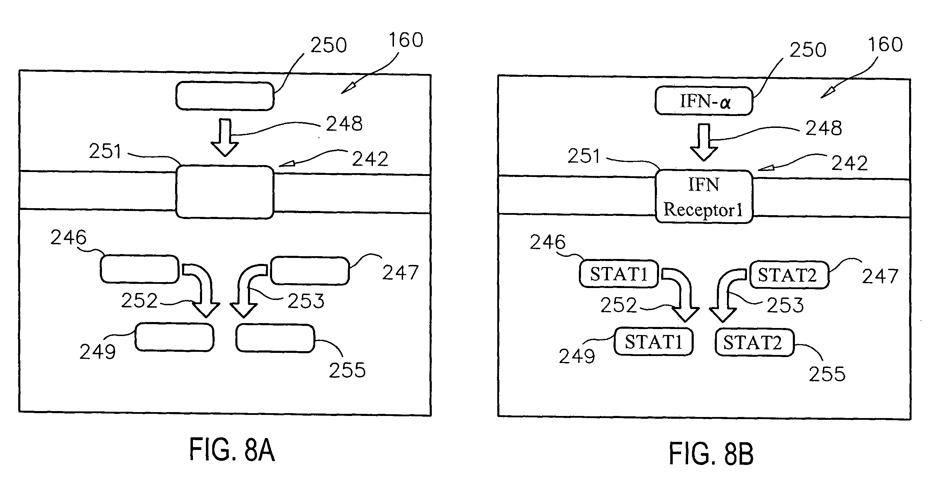

Stencils can also be used effectively as a query interface for a knowledge base. One example of this would be to form a query out of a partially assigned stencil. When this query is submitted, the knowledge base, in response, returns a set of data that constitutes all valid completions of the unassigned stencil elements. In this way, a user may use stencils to graphically form a query such as "find all receptors for pathways involving the PI3-kinase protein complex", for example by partially filling in the stencil in Fig. 8A by assigning PI3-kinase protein complex to entry 246.

As noted above, a user can assign biological entities to stencils. When the assignments are made, this information is automatically added to the local format, effectively mapping the stencil elements to data in the local format. Thus, using stencils is a way of graphically adding metadata to other structured and unstructured data. Stencils are fully annotatable and there are a number of ways in which annotation can be made. Annotations may be input manually, such as by the user typing them into the stencil. For example, in network editor 200 (Fig. 3), double-clicking on stencil 160a or 160b will result in an editable "property sheet" for a stencil being displayed (not shown), in which annotations can be typed in. Further, annotations may be made by data mining and inference tools that generate the annotation.

Fig. 6 shows two examples of stencils 160. These stencils are used to represent simple promotion and binding relationships, as noted in the filled in versions of stencils 160 on the right hand side of Fig. 6. The information used to fill in stencils 160 may be derived via knowledge extraction from the scientific literature, as noted. The top stencil 160 in Fig. 6 shows empty and filled stencils representing the interaction "IL-1-α induces the proliferation of Th2 cells." The bottom stencil 160 shows an example where an interaction itself (i.e., stencil 160 from the top of Fig. 6) serves as an entity in another interaction. This corresponds to the interaction expressed in the text "The binding of TNF1-α to the IL-β receptor promotes the expression of IGL genes."

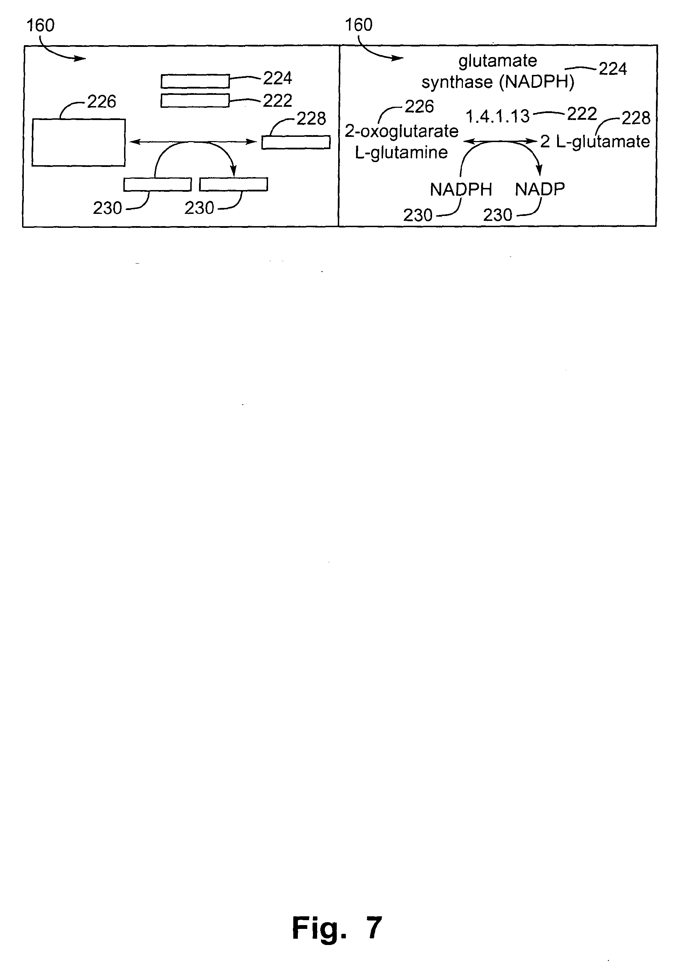

Another example of a common biological relationship is a reaction, which includes substrates, products, catalysts, and co-factors, as well as directionality information. Fig. 7 shows an example of empty and filled-in stencils 160 for an example reaction. The blank stencil 160 in Fig. 7 includes symbols for a particular type of reaction which includes symbols for reaction (e.g., rectangle 222), catalyst (e.g., rectangle 224), and substrates, products, and co-factors (e.g., rectangles 226, 228 and 230, respectively). The "nouns" of the relationship are the substrates, products, co-factors, and catalysts. The "verb" is the reaction. The right side of Fig. 7 shows the stencil after being "filled in" by the user, with substrates 226 (2-oxoglutarate and L-glutamine), product 228 (2 L-glutamate), catalyst 224 (glutamate synthase (NADPH)), co-factors 230 (NADPH and NADP) and reaction 222 (represented by the bi-directional arrows and identified by the Enzyme Commission (EC) number 1.4.1.1.3). Note that the double-headed arrow between substrates and product indicates that this is a reversible reaction.

Figs. 8A and 8B show an example of a stencil used in forming what is commonly referred to as a "cartoon diagram". The stencil 160 shown is for a cartoon diagram representing a biological process in the cell, such as a signal transduction pathway. Fig. 8A shows the empty stencil 160, while Fig. 8B shows a filled-in stencil 160 that represents a signal transduction sub-path, containing the binding of a ligand to a receptor on a cell membrane and a small number of subsequent cascading activation events.

The "nouns" (or entities) of the interaction are the ligand (IFN-α) 250, the receptor (IFN Receptor1) 242, the secondary messenger proteins (STAT1) 246 and (STAT2) 247, and a protein complex consisting of STAT1 249 and STAT2 255. The "verbs" (or interactions) are the arrows 248, 252 and 253, which in this example represent binding actions. Note that in this example, stencil 160 also represents the context of "locale" within a cell, in this case the cell membrane. Thus, stencils enable cellular localization to be used as an organizing principle. This may be useful in parsing diagrams programmatically to find possible points of interest.

Using the present invention, a user may also design and save new stencils according to his or her own needs. Existing stencils 160 can be used as building blocks and can be extended by adding graphical primitives, such as shapes, lines, arrows, arcs, and/or text. New stencils can also be built from scratch using graphical primitives. Multiple stencils can be merged, either by merging them around a common element or by connecting them with graphical primitives. An example of merging is where the product of one reaction stencil serves as one of the substrates or components for a second stencil (as shown in Figs. 5A and 5B, for example).

Stencils may be built up hierarchically; that is a stencil can contain other stencils as well as primitive elements. In this way, a user can build up larger diagrams from smaller pieces. Likewise, the smaller pieces of a diagram can be split off and worked upon separately.

Diagrams may also be imported from external sources (including, but not limited to BioCarta or KEGG pathways) and used for building stencils. One technique for doing so is to attach additional stencil elements to an imported diagram. Another approach is to break off sub-parts of one or more imported diagrams and form stencils from them. Automated tools for searching existing biological diagrams may be employed to identify pre-defined stencil formats. Diagrams containing the desired pre-defined stencil formats may then be imported to the system, wherein the system is then used to decompose a larger representation into its building blocks (stencils). When working with an imported diagram, the user can keep specific nodes as they are or make them "empty". For example, one might import a diagram of a signal transduction pathway, such as the sub-path shown in Figs. 5A and 5B. The user may wish to keep the receptor element as is, i.e. as Type I receptor 167, but perhaps make the other elements empty. The new stencil thus created would always have a Type I receptor, while other elements would be assignable.

Stencils may be used and combined to make connections across multiple levels of biological abstraction. For example, one may connect a stencil 160 that represents a biochemical reaction to a stencil 160 that represents a physiological process or disease process. The resulting structure assists the user to visualize and reason across multiple levels of abstraction.

Another feature of the present invention compares two or more stencils for structural differences, using graph theoretic methods. This may involve analyzing and comparing graph properties, such as shortest-path, minimum spanning tree, and/or graph order and size, for example.

Another feature enables the user to merge stencils, to allow, for example, merging a stencil that represents part of a species-specific pathway with a canonical pathway. This feature also supports collaboration, enabling different users to merge related stencils created by one another.

In addition to the advantages of stencil-based model definition for knowledge extraction approaches, there are other applications for which the stencil approach provides benefits. As mentioned above, there are tools for modeling and simulating, both qualitatively and quantitatively, the global response of a biological system to a stimulus, treatment, or condition. These are often referred to collectively as in silico modeling and simulation tools. Such tools require a detailed model of biological entities and the connections and interactions between them. To this end, some of these systems provide graphical "network editing" tools as a modeling interface to simulation. Building up networks from single elements can be tedious and error prone. A stencil-based approach for network editing can provide a set of "building blocks" for constructing biological networks, which make it easier and less error prone to compose and capture the semantics desired by the simulation environment.

It may also be useful to attach stencils, via the local format, to other kinds of data, such as mass spectra or documents from the scientific literature, for example. Attachment to mass spectra or other data provides a rich form of annotation for such detailed data, contextualizing that data graphically. Attachment to scientific literature facilitates a form of graphical "notetaking", where the gist of a document can be captured by one or more stencils or diagrams. It is often the case that researchers mark up textual documents with diagrams as a form of summarization. Stencils provide way to accomplish this task in the digital domain, where such notes and summarizations are retrievable and computable.

Automatic or machine construction of biological diagrams may be performed by inferring stencil structures from experimental data. The experimental data may be processed by algorithms that infer network structure from experimental data profiles, such as gene expression profiles, and the network structure represented by local format objects. An example algorithm that infers network structure from experimental gene expression data is described in Friedman, N., et al, Using Bayesian Networks to Analyze Expression Data, *Journal of Computational Biology,* 7:601-620, 2000, which is incorporated herein, in its entirety, by reference thereto. Local format objects may be searched for sub-graphs that map to stencils in a manner similar to that described in Shen-Orr, S. et al, Network Motifs in the Transcriptional Regulation Network of Escherichia coli, *Nature Genetics,* 2002. A network of local format objects may be represented by a connectivity matrix. Possible combinations of sub-matrices of three and four nodes each are tested for equivalence with stencils in the library, as described above.

Expression patterns representative of biological entities may be compared for similarity to infer existing relationships between the entities. The expression patterns, for example may be measures of differential quantities of biological entities relative to at least one reference sample, e.g., gene expression data, protein abundance data, metabolite abundance data, or other measure of differential quantity of a biological entity versus a reference sample. A pattern or expression for each biological entity may be derived from a multiplicity of measurements of expression values over varying conditions.

Similarity determinations may be based upon application of a distance metric, such as squared Euclidean distance, Pearson correlation coefficient, or the like. A numerical similarity threshold distance metric may be applied to determine whether any particular distance measurement is determined to be "similar" or not. The similarity measurements that are determined to be similar, i.e., within the bounds of the similarity threshold distance metric, may be considered to be co-regulated and, by implication, related in a biological interaction.

The similarity measurements having been determined to meet the similarity threshold may be further assessed for statistical significance, that is to determine the likelihood that true similarity exists versus the likelihood of a random occurrence. Typical tests that are used to make such as determination include, but are not limited to the *t* test, and the Analysis of Variance (ANOVA) test. However, other tests for carrying out this determination would be readily apparent to those skilled in the statistics arts.

A set of statistically significant biological interactions (implied by statistically significant similarity measurements among expression patterns) may then be merged together, wherein duplicate biological entities (are represented by the expression patterns) are joined together to form nodes in a resulting biological network. Such biological networks may be examined for patterns of entities and interactions that appear considerably more frequently than in random networks. The frequently occurring patterns may be matched against elements in a library of stencils to identify matching frequently occurring patterns with existing stencils.

Stencils support parsing existing biological visualizations (using the local format) and assigning existing stencils from the stencil library to matches in the existing biological diagram having been converted to the local format, to construct the diagram using stencils. Implicit rule checking is built into the stencils to facilitate the matching.

Conversely, a user may parse existing biological visualizations, or a document or corpus of documents, and receive a set of recognized stencils as a result of the query.

To facilitate overview and navigation, the set of stencils can be shown in a "spreadsheet viewer" visualization, such as described in co-pending commonly owned Application Serial No. 10/641,492

All stencils shown in cells of the spreadsheet viewer may be linked back to original source.

As described above, the present invention offers stencil-based analysis and information retrieval tools to perform functions such as searching textual documents for filled or unfilled stencils, using the local format; querying experimental data to find matches to one or more stencils; querying existing biological diagrams, based upon a user's context of one or more selected stencils, and displaying any portions of the existing biological diagram that match any stencil in the user's context; and/or querying a set of local format objects to find one or more stencils in the user's context.

Stencils facilitate user-guidance of knowledge extraction tools, addressing the problem of disambiguation/causality determination. Although it is currently possible to identify interactions between biological entities from textual documents, for example, using automated text mining tools, (e.g., it is possible to identify the "nouns" and "verbs" used in describing an interaction involving entities), it was not heretofore possible to unambiguously identify causality or directionality of the interactions. A method and system for user-guided knowledge extraction is described in co-pending commonly owned Application Serial No. 10/154,524, which was incorporated by reference above. Described are methods and systems wherein automated text mining techniques are used to extract "nouns" (e.g. biological entities) and "verbs" (e.g. relationships) from sentences in scientific text. Thus, knowledge extraction from scientific literature, e.g. via text mining, can identify biological entities that are involved in a relationship, for example a promotion interaction involving two genes. The resulting interpretation is represented in a restricted grammar, referred to as "local format", which was described above.

The present invention extends the functionality and versatility of the local format by augmenting automated tools to enable the user to interact with the processes to clarify and/or correct the results of the process by disambiguation, and to employ higher level tools, such as stencils, for automatic construction of, and interaction with, biological diagrams. In the current invention, stencils may be functionally implemented in the graphical pane 150 of the text viewer 100 described in Application Serial No. 10/642,376 (e.g., see Figs. 4A and 4B).

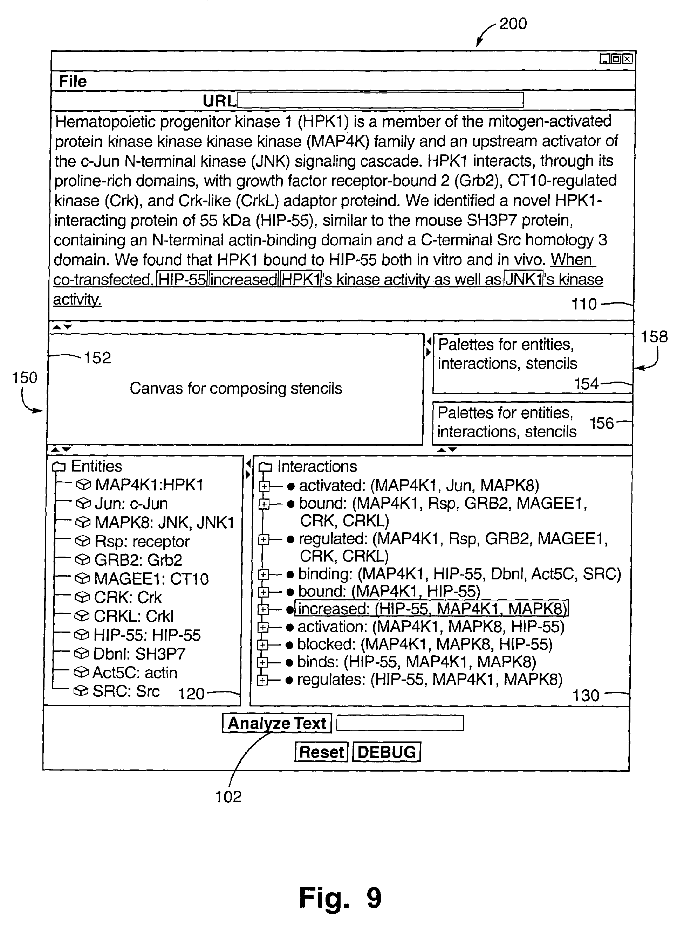

Fig. 9 shows an example of an application tool in which stencils are used to help in disambiguating directionality of interactions extracted from scientific literature and incorporated into a text viewer.

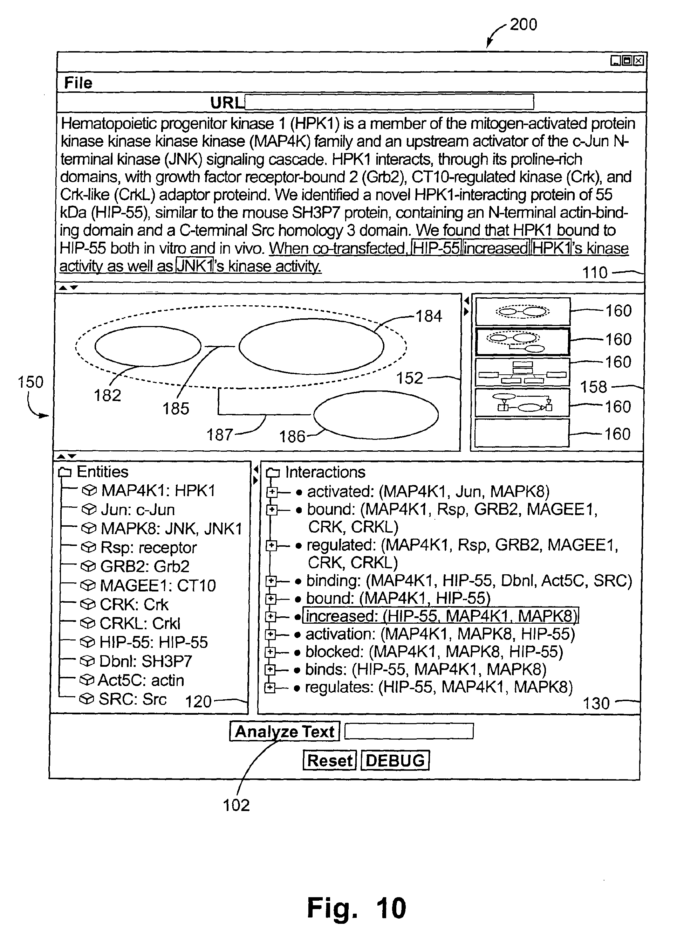

Fig. 10 shows tool 200 in which a plurality of pre-designed empty stencils 160 are provided in palette 158 for use in constructing filled in or populated stencils to represent unambiguous interactions. In this example, the user has entered textual material into window 110 in a manner as described in Application 10/642,376 and has selected the "Analyze Text" button 102 to populate windows 120 and 130, respectively, with the entities and interactions discovered in the text. However, instead of selecting and working with an existing interaction, the user decides to create a new interaction, using the extracted entities and choosing from a palette of pre-defined stencils 160. The user performs a gesture (could be right-mouse menu select or a button press) and a palette 158 appears. Palette 158 is populated with "empty" stencils, as shown in Fig. 10.

Although canvas 152 is initially blank, Fig. 10 shows a state of progression where the user has selected the second from the top stencil 160 (shown by the highlighted box around the stencil), which causes canvas 152 to be automatically populated with this blank or empty stencil. Alternatively, this embodiment may be configured so that the user populates the canvas 152 by dragging and dropping stencil 160 from palette 158 to canvas 152. Either way, this results in an "empty" interaction on canvas 152 that can now be assigned "affecters", "affecteds", and directionality. Additionally, or alternatively, the user may add elements to the canvas via free-hand "sketching" of shapes.

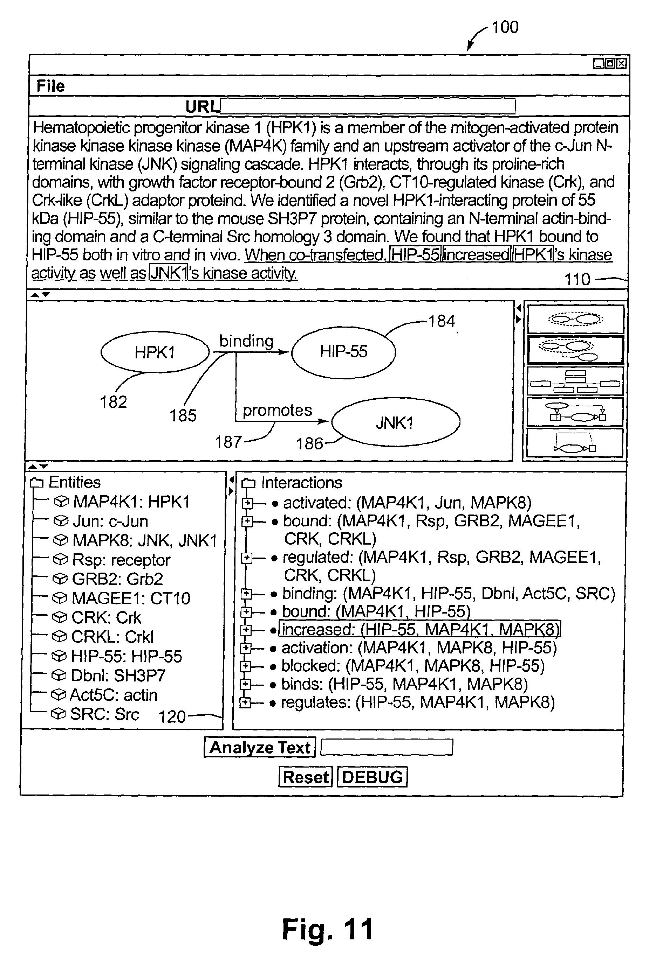

In this example, the user populates stencil 160 by dragging and dropping affecter(s) and affected(s) by dragging and dropping entities from the "Entities" list 120 into the shapes 182,184,186 (e.g., lavender colored ovals) in stencil 160. The user can also assign directionality to the interaction by gesturing (perhaps via a select and right-mouse menu combination or by dragging the mouse along the lines 185,187 (which may also be color coded, e.g., red and blue, respectively) lines in a stroking gesture. The user can also associate textual descriptions with the interaction by dragging and dropping text from the text window 110 onto components of stencil 160. The result of these actions is shown in Fig. 11. The result is an unambiguous, graphical description of one or more interactions (in this example, two interactions are described) described in the literature, with annotations included in the graphical description in the form of meta-data.

Stencils may be filled-in by the user, using the techniques described above, to define a user context, for use in information extraction, as described in more detail in co-pending, commonly owned Application Serial No. 10/642,376 Alternatively, the user may define the user context with one or more blank stencils, or a combination of blank and filled-in stencils.

New stencils can be created by a user via a Stencils Manager that is associated with the graphical network editor described in co-pending commonly owned Application Serial No. 10/641,492 which was incorporated by reference above. A subset of nodes and links in a diagram may be selected, via lassoing with mouse or cntl-click mouse operations. The selected subset may be designated as a new stencil. The system will prompt the user for a name for the stencil and then will construct the new stencil, drawing from information in the selected nodes and links. For each node in the diagram, there is a corresponding slot in the stencil. The slot will be able to be filled in with any entity that matches the type of the local format object in a corresponding diagram node. For example, if the local format object is a protein, then the corresponding slot of the stencil will accept any protein as its value. The user may determine the level of specificity that a slot enforces. For example, if the local format object is a MAP-kinase protein, which is an enzyme, which is a protein, the user can choose whether the corresponding slot will accept only MAP-kinase proteins, or all enzymes, or all proteins. Local format interactions will map on a one-to-one basis to the corresponding interactions in the new stencil. For example, a "promote" interaction in the local format will be mapped into a "promote" relation in the corresponding slot of the stencil.

Similarly, the user may determine the level of specificity that a slot for an interaction enforces. For example, if the local format object is "promote", as in the example above, then "promote by phosphorylation" or "promote by methylation", each of which are promotion interactions, may be inserted. However, the user may choose whether the corresponding slot will accept only "promote by phosphorylation" promotions for example, or may limit the slot to some other more specific subset of "promotions", or may choose to accept any "promotion" generally.

The Stencils Manager also enables the user to modify, copy, and delete stencils. These operations are accomplished via graphical editing methods, in a manner that will be apparent to those persons skilled in the art. When a stencil is modified or deleted, those local format objects that had been created using the stencil are not modified or deleted, however. Once a stencil is instantiated to form new local format objects, that instantiation exists on its own, separately from the stencil used to create it.

New stencils can be inferred from graphical structures when certain patterns of nodes and links appear considerably more frequently than in random networks. A graphical structure may be converted into local format objects in a manner described in co-pending commonly owned Application Serial No. 10/641,492 Local format objects may then be searched for sub-graphs having frequently occurring patterns and/or nodes and links in a manner similar to that described in Shen-Orr, S. et al, Network Motifs in the Transcriptional Regulation Network of Escherichia coli, *Nature Genetics,* 2002. The network of local format objects can be represented by a connectivity matrix. Possible combinations of sub-matrices of three and four nodes each are tested for their frequency of appearance in said graphical structure, in comparison to the frequency of appearance of such combination in a randomized version of the graphical structure. The randomization of the graphical structure may be accomplished in a manner similar to that described in Shen-Orr, S. et al, Network Motifs in the Transcriptional Regulation Network of Escherichia coli, *Nature Genetics,* 2002. A frequently occurring combination, when identified, may be designated as a new stencil. The system may prompt the user for a name for the stencil and then construct the new stencil, drawing from information in the corresponding nodes and links in the diagram. For each node in the diagram, there is a corresponding slot in the stencil. The slot will be able to be filled in with any entity that matches the type of the local format object in corresponding diagram node, as described above. Specificity determinations for both entity and interaction variables may optionally be set by the user, as described above.

Using the above described principles, techniques and systems, stencils may be employed as a validation and/or inference aid to convert unstructured data, for example through disambiguation of textual information mined from textual documents and/or by setting the user's context for specific knowledge to be extracted from textual documents and used to populate stencils.

The network construction techniques described (e.g., construction of graphical diagrams using stencils) may be used to provide user defined biological networks for knowledge representation, documentation, and/or notetaking.

The present invention facilitates complexity management by providing higher levels of abstraction (i.e., stencils) than an unstructured collection of "atomic" elements such as genes, proteins, etc. Further, stencils not only organize and disambiguate relationships between entities and represent them in a higher level representation, but do so in a manner that is familiar and intuitive to the user (i.e., graphically).

Stencils also provide a consistency of representation for commonly used biological constructs, such as phosphorylation. Thus, in comparison to working at the level of individual entities and interactions, the use of stencils can reduce errors in constructing and documenting biological entities, because equivalent information is represented in an equivalent way throughout the network.

Because stencils can be annotated and linked to other forms of structured data, stencils provide a multi-dimensional interaction or linkage between the stencil and heterogeneous data.

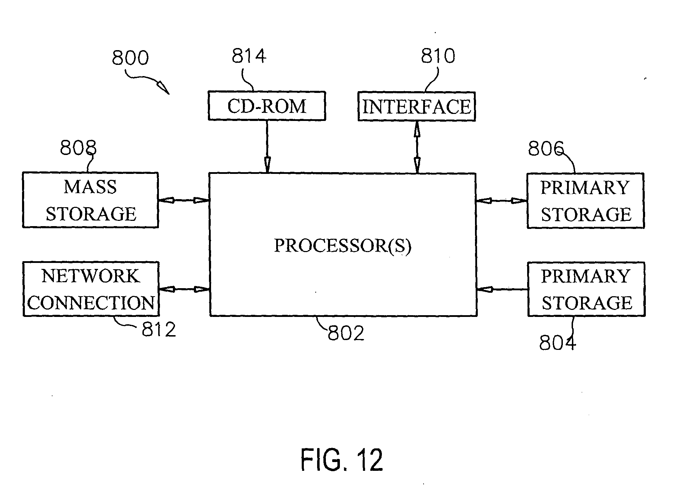

Fig. 12 illustrates a typical computer system in accordance with an embodiment of the present invention. The computer system 800 includes any number of processors 802 (also referred to as central processing units, or CPUs) that are coupled to storage devices including primary storage 806 (typically a random access memory, or RAM), primary storage 804 (typically a read only memory, or ROM). As is well known in the art, primary storage 804 acts to transfer data and instructions uni-directionally to the CPU and primary storage 806 is used typically to transfer data and instructions in a bi-directional manner Both of these primary storage devices may include any suitable computer-readable media such as those described above. A mass storage device 808 is also coupled bi-directionally to CPU 802 and provides additional data storage capacity and may include any of the computer-readable media described above. Mass storage device 808 may be used to store programs, data and the like and is typically a secondary storage medium such as a hard disk that is slower than primary storage. It will be appreciated that the information retained within the mass storage device 808, may, in appropriate cases, be incorporated in standard fashion as part of primary storage 806 as virtual memory. A specific mass storage device such as a CD-ROM 814 may also pass data uni-directionally to the CPU.

CPU 802 is also coupled to an interface 810 that includes one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Finally, CPU 802 optionally may be coupled to a computer or telecommunications network using a network connection as shown generally at 812. With such a network connection, it is contemplated that the CPU might receive information from the network, or might output information to the network in the course of performing the above-described method steps. The above-described devices and materials will be familiar to those of skill in the computer hardware and software arts.

The hardware elements described above may implement the instructions of multiple software modules for performing the operations of this invention. For example, instructions for population of stencils may be stored on mass storage device 808 or 814 and executed on CPU 808 in conjunction with primary memory 806.

In addition, embodiments of the present invention further relate to computer readable media or computer program products that include program instructions and/or data (including data structures) for performing various computer-implemented operations. The media and program instructions may be those specially designed and constructed for the purposes of the present invention, or they may be of the kind well known and available to those having skill in the computer software arts. Examples of computer-readable media include, but are not limited to, magnetic media such as hard disks, floppy disks, and magnetic tape; optical media such as CD-ROM, CDRW, DVD-ROM, or DVD-RW disks; magneto-optical media such as floptical disks; and hardware devices that are specially configured to store and perform program instructions, such as read-only memory devices (ROM) and random access memory (RAM). Examples of program instructions include both machine code, such as produced by a compiler, and files containing higher level code that may be executed by the computer using an interpreter.

While the present invention has been described with reference to the specific embodiments thereof, it should be understood by those skilled in the art that various changes may be made and equivalents may be substituted without departing from the true scope of the invention. In addition, many modifications may be made to adapt a particular model, tool, process, process step or steps, to the objective and scope of the present invention. All such modifications are intended to be within the scope of the claims appended hereto.

## Claims

1. A system for manipulating biological data comprising:
a library of re-usable stencils (160) for representing biological interactions (137, 138, 139, 156, 157);
means for selecting stencils (160) to be populated with specific biological information;
means for assigning specific biological data to selected stencils (160); and
means for displaying stencils (160) with the assigned specific biological data.

2. The system of claim 1, further comprising means for connecting common elements of said stencils (160) with assigned specific biological data to display a biological diagram having said stencils (160) as components thereof.

3. The system of claim 1 or 2, further comprising means for designing and saving additional stencils (160) not previously contained in said library.

4. The system of any of claims 1-3, further comprising means for designing and associating rules with said stencils (160); and means for rule checking said rules to validate an interaction (137, 138, 139, 156, 157) represented by a stencil containing specific biological data.

5. The system of claim 4, further comprising means for rule checking said rules against additional data.

6. The system of any of claims 1-5, further comprising means for navigating to data selected from said specific biological data and displayed on at least one of said stencils (160).

7. The system of any of claims 1-6, further comprising means for comparing, among two or more selected stencils (160), specific data assigned thereto and displaying results of said comparison.

8. The system of claim 7, further comprising means for mapping between said selected stencils (160) containing specific biological data and an existing biological diagram.

9. The system of any of claims 1-8, further comprising means for adding elements to a stencil on a canvas or creating a stencil on the canvas by freehand sketching by the user.

10. The system of any of claims 1-9, further comprising means for merging said stencils (160) with a biological network and means for displaying said stencils (160) merged with said biological network.

11. The system of any of claims 1-10, further comprising means for linking the displayed stencils (160) with other sources of biological data from which the specific biological data was extracted, using a local formatting language.

12. The system of any of claims 1-10, further comprising:
a text viewer (100) into which at least a portion of a textual document may be imported and viewed;
means for text mining the at least a portion of a textual document having been imported into the text viewer;
a list-based text editor that lists entities (130, 167, 169, 170) and interactions (137, 138, 139, 156, 157) having been identified by said means for text mining;
a canvas area (152) for generating biological diagrams; and
means for populating said stencils (160) on said canvas with one or more of said entities (130, 167, 169, 170) and interactions (137, 138, 139, 156, 157) identified by said means for text mining.

13. The system of claim 12, further comprising:
means for assigning directionality to the listed interactions (137, 138, 139, 156, 157); and
means for selecting interactions (137, 138, 139, 156, 157) and associated entities (130, 167, 169, 170) in the list-based editor, merging common entities (130, 167, 169, 170) and displaying a resulting network of the interactions (137, 138, 139, 156, 157) in said network viewer;
wherein said entities (130, 167, 169, 170) and associated entities (130, 167, 169, 170) are displayed visually using said stencils (160) that include display of directionality, and wherein said resulting network comprises a plurality of merged stencils (160).

14. The system of any of claims 1-13, further comprising:
a diagram viewer and means for importing at least a portion of an existing static or dynamic biological diagram into said diagram viewer;
means for overlaying at least one of said stencils (160) having been populated by at least one of biological concepts, entities (130, 167, 169, 170), interactions (137, 138, 139, 156, 157) and relationships on said at least a portion of an existing biological diagram that is displayed in said diagram viewer;
and means for visually distinguishing the overlaid stencils (160) from a remainder of the displayed biological diagram.
